# EUROPEAN PATENT SPECIFICATION

(11) **EP 2 327 780 B1**
(45) Date of publication and mention of the grant of the patent: **13.02.2013**
(21) Application number: 10184627.7
(22) Date of filing: 23.12.2005
(51) Int. Cl.: C12N 15/10, G01N 33/68

(54) **Method for screening and selecting ligands**
Verfahren zum Screening und Auswahl von Liganden
Procédé pour le tri et la séléction de ligands

(30) Priority: 23.12.2004 EP 04447298
(43) Date of publication of application: 01.06.2011
(62) Divisional of application: 05824968.1
(73) Proprietor: Vrije Universiteit Brussel, 1050 Brussel (BE); Ministerie van Landsverdediging, 1000 Brussels (BE)
(72) Inventor: Zizi, Martin, 1350 Enines (BE); Roman, Inge, 9660 Brakel (BE)
(74) Representative: pronovem

(56) References cited:
- WO-A1-2005/010532
- SCHIER R ET AL: "EFFICIENT IN VITRO AFFINITY MATURATION OF PHAGE ANTIBODIES USING BIACORE GUIDED SELECTIONS", HUMAN ANTIBODIES AND HYBRIDOMAS, vol. 7, no. 3, 1996, pages 97-105, XP001002220,
- SHANK-RETZLAFF MARY L ET AL: "Analyte gradien-surface plasmon resonance: a one-step method for determining kinetics tares and macromolecular binding affinities", ANALYTICAL CHEMISTRY, AMERICAN CHEMICAL SOCIETY, US, vol. 72, no. 17, 1 September 2000 (2000-09-01), pages 4212-4220, XP002994633, ISSN: 0003-2700, DOI: DOI:10.1021/AC0001030
- DATABASE BIOSIS [Online] BIOSCIENCES INFORMATION SERVICE, PHILADELPHIA, PA, US; January 2004 (2004-01), ROMAN INGE ET AL: "A systematic search for physiologically relevant ligands of the mitochondrial membrane protein VDAC.", XP002335826, Database accession no. PREV200400136899

## Description

### FIELD OF THE INVENTION

The present invention relates to a new method for screening, selecting and identifying ligands of a membrane protein.

### BACKGROUND

The completion of the human genome sequencing paved the way for the study of proteomes i.e. the context-dependent interrelationships between the sets of expressed proteins in a given cell type.

A finer description of all these protein-protein interactions will lead to a better understanding of most patho-physiological conditions and to an improved pharmacological and therapeutical control of the relevant cellular processes.

Because of the sheer volume of the task, finding new ligands interfering with cellular proteins cannot be based on the classical trial and error approach to improve naturally occurring molecules.

Over the years, several new approaches have been developed including theoretical ab initio methods, computer-aided in silico design and combinatorial chemistry. The latter allows the selection of the relevant molecules from a huge molecular library by a screening process.

Various methodologies have been developed but compared to other available proteomics screening tools the phage display methodology offers two major advantages in the study of protein-protein interactions. It combines the full power of combinatorial chemistry -as in chemical dynamic libraries- with an in vitro Darwinian evolution as phages compete with each other through experimentally defined selection and amplification processes.

Bacteriophages allow to present -and thus screen- entire libraries of epitopes on their surface as fusion proteins.

Selection can be obtained by (bio)panning the combinatorial phage populations against specific molecular targets, and amplification is performed by infecting pili (+) host bacteria with the selected phages, and thus consequently seeding a second generation of a combinatorial phage population. Such process is called a round and can be iterated.

Membrane proteins however remained a frontier as the solubilized protein conformer usable for the biopanning rounds may not display the correct folds.

Another problem lies with poor results while panning against membrane proteins. During the selection process one is confronted with the fact that the selection for relatively hydrophobic determinants interacting with the membrane protein has to be achieved against the hydrophobicity of the experimental plasticware used during the panning experiments i.e. one gets chemical selection for other compounds than for the target protein. Therefore relative selectivity of panning results against membrane protein as measured by ELISA is never very high.

### SUMMARY OF THE INVENTION

The present invention provides a method for screening and selection of/for ligands, wherein the target is immobilized on a suitable support, and is any molecule susceptible of binding with said ligands, comprising the step of providing conditions of mass-transfer-limitation, wherein the ligand availability is limited with respect to the target by working under the diffusion limit and wherein the said mass-transfer-limitation conditions are provided by a continuous flow of ligands over this immobilized target.

Advantageously, in the method according to the invention, these conditions of mass-transfer-limitation are provided during the biopanning round(s).

In the method according to the invention these mass-transfer-limited conditions are preferably further provided:
- by raising the mass and/or volume of the ligands, preferably in phage display technology or by immobilizing the ligands on beads; and /or
- by providing a magnetic field, this magnetic field being preferably direct current magnetic field or an alternate current magnetic field; and/or
- by providing a temperature gradient.

Preferably, in the method according to the invention, the ligands are any molecules or macro-molecules, preferably sugars, proteins, antigens, antibodies, enzymes, DNA, RNA, hormones, neurotransmitters, cells, viruses or any biological entities. More preferably, these ligands are phage-displayed peptides.

In the method according to the invention, the target is preferably a receptor susceptible of binding with these ligands. Preferably, this receptor is incorporated into liposomes or is immobilized upon a sensor chip.

Another aspect of the present invention is related to the use of the method according to the invention for drug design, for catalyst design, for trapping-ligand design or for molecular scavenger design.

Another aspect of the present invention is related to the method according to the invention comprising the steps of:
i. preparing a solution containing a library of phage-displayed peptides to be screened,
ii. immobilizing the target membrane protein incorporated into liposomes upon a sensor chip,
iii. perfusing a surface plasmon detector device with this solution, for contacting this phage displayed peptides with this target captured upon, this sensor chip via these liposomes,
iv. eluting the phage displayed peptides, and optionally, repeating, at least once,
   - step (i) wherein said solution is any amplified eluted fraction(s) of previous step (iv),
   - optionally step (ii),
   - step (iii), and
   - step (iv).

Preferably, in a method of the invention, at least one flow cell is used to minimize binding.

Advantageously, in a method of the invention, the perfusing step is performed at a flow rate as low as technically possible, in particular can be comprised between about 3 and about 0.5 ml/min.

### DETAILED DESCRIPTION OF THE INVENTION

The present invention provides a method for screening of ligands wherein the target is a membrane protein, which is reconstituted into a membrane environment, using the surface plasmon resonance (SPR) technology.

A method according to the invention, as defined in claim 13, comprises the steps of:
i. preparing a solution containing a library of phage-displayed peptides to be screened,
ii. immobilizing the target membrane protein incorporated into liposomes upon a sensor chip,
iii. perfusing a surface plasmon detector (SPR) device with said solution, for contacting said phage displayed peptides with said target captured upon said sensor chip via said liposomes,
iv. eluting the phage displayed peptides, and
v. optionally, repeating, at least once:
   - step (i) wherein said solution is any amplified eluted fraction(s) of previous step (iv),
   - optionally step (ii),
   - step (iii), and
   - step (iv).

Advantageously, the eluting step further comprises a fractionating step resulting in different fractions being collected.

Depending on the SPR device use, there may be different flow cells, e. g. four different flow cells, upon each sensor chip.

Preferably, in a method of the invention, at least one flow cell is used to minimize the aspecific binding.

Advantageously, in a method of the invention, the perfusing step is performed at a flow rate as low as technically possible, in particular can be comprised between about 3 and about 0,5 µl/min.

Advantageously, in a method of the invention, the reiteration in step (v) can be twice, preferably three times, more preferably four times.

Advantageously, in a method of the invention, the library of phage-displayed peptides is from any naturally occurring or artificial nucleic acids library.

In a preferred method of the invention, the target is VDAC.

The object of the present invention is a method for screening of/for ligands, wherein the target is any molecule or macro-molecule susceptible of binding with said ligands, comprising the step of providing conditions where availability of potential binders becomes limited (mass transfer limited conditions also referred to as mass transport limited conditions, abbreviated MTL).

The MTL conditions to be used in a method of the invention are to be opposed to the diffusion-limited conditions (by reference to the known concept of Smoluchovski's limit and equation) that are used in prior art screening methods. Indeed, in prior art screening methods, the conditions sought are those wherein the concentration and diffusion of the ligands within the solution are optimal (also referred to as diffusion-limited conditions i.e. conditions where the binding reaction is not limited by the availability of the potential binders but by their free diffusion in the solution).

To the contrary, in a MTL screening method of the invention, the conditions sought are those wherein the diffusion (and possibly (optionally) the availability) of the ligands is (are) not allowed to be optimal from a kinetic point of view.

In a MTL screening method of the invention, the MTL conditions can result from any suitable means such that the diffusion-limited conditions are not met.

It should be appreciated that any means, known by the skilled person, for providing MTL conditions can be used in a MTL screening method of the invention.

For example, the MTL conditions can be generated by any suitable means that affect the diffusion coefficient of the ligands.

For example, by raising the mass and/or volume of the ligands (e.g. in phage display technology, or by attaching the ligands on beads, etc.), and/or by providing a magnetic field (direct current or alternate current magnetic field), and/or by providing a temperature gradient, etc. (i.e. any suitable condition(s) for hindering steady-state kinetics are envisaged).

Preferably, the MTL conditions result from a continuous flow of the solution containing the binders/ligands to be screened.

Said continuous flow can be combined with at least one other means, such as the mass, the volume, the temperature, the magnetic field, the density of the solution used, etc.

In a MTL screening method of the invention, the target is immobilized on any suitable surface or support.

In a MTL screening method of the invention, a ligand can be any molecule or macro-molecule.

More particularly, a ligand can be a sugar, a protein, an antigen, an antibody, an enzyme, a DNA, a RNA, an hormone, a neurotransmitter, a cell, a virus or any biological entity.

Advantageously, a MTL screening method of the invention can be used for drug design.

More particularly, a MTL screening method of the invention can be used for selecting physiologically expressed peptides that bind to a target.

It may be used in combination with the surface plasmon resonance (SPR) technology.

More particularly, a MTL screening method of the invention can be used for selecting physiologically expressed peptides that bind to a membrane protein incorporated into liposomes.

Advantageously, said liposomes are immobilized upon a sensor chip (regardless of the technical nature of its output reading methodology).

Advantageously, said peptides are phage-displayed peptides and the target is a receptor susceptible of binding with said peptides.

Advantageously, a MTL screening method of the invention can be used for catalyst design.

Advantageously, a MTL screening method of the invention can be used for trapping-ligand design, or for scavenger design, in particular for environmental or ecological applications, related for example to air,pollution, water quality, solid or liquid waste, global warming, etc.

In the context of the present invention, the term "phage-displayed peptides" refers to any molecule of two, 5, 10, 20, 50, 100, 200, 500 or more amino acids linked by peptide bonds, notably peptides, polypeptides, protein, oligomers, etc., displayed by bacteriophages, resulting from the expression of a nucleic acid of any origin, i.e. natural-occurring or artificial nucleic acid.

The term "peptides" may also be referred herein to as "epitopes".

In the context of the present invention, the term "phages solution", e.g. the solution prepared in step (i) of a method of the invention, may also be referred to as a "sample".

In the context of the present invention, the term "flow cell(s)" can also refer to "flow channel(s)", or to "chamber(s)".

The present invention provides a method for selecting physiologically expressed peptides that bind to a membrane protein close to its native conformation.

A method according to the invention combines the phage display methodology with the surface plasmon resonance (SPR) technology.

A method according to the invention, as defined in claim 13, comprises the steps of:
i. preparing a solution containing a library of phage-displayed peptides to be screened,
ii. immobilizing the target membrane protein incorporated into liposomes upon a sensor chip,
iii. perfusing a surface plasmon detector (SPR) device with said solution, for contacting said phage displayed peptides with said target captured upon said sensor chip via said liposomes,
iv. eluting the phage displayed peptides, and
v. optionally, repeating, at, least, once:
   - step (i) wherein said solution is any amplified eluted fraction(s) of previous step (iv),
   - optionally step (ii),
   - step (iii), and
   - step (iv).

In particular, a method according to the invention, as defined in claim 13, comprises the steps of:
i. preparing a solution containing a library of phage-displayed peptides to be screened,
ii. immobilizing the target membrane protein incorporated into liposomes upon at least one of the flow cells of a sensor chip,
iii. flowing said prepared solution over said flow cells of said sensor chip,
iv. eluting the phage displayed peptides, and
v. optionally, repeating at least once, step (i), wherein said solution is any amplified eluted fraction (s) of previous step (iv), optionally step (ii), step (iii) and step (iv).

In the context of the present invention, the steps (iii) and (iv) are performed inside the chamber of a SPR biosensor device, e.g. Biacore® 2000 device, and can also be referred to as a (bio)panning round.

In particular, the (bio)panning rounds may consist of or comprise the step of contacting a library of phage-displayed peptides with the target membrane protein embedded in liposomes immobilized upon a sensor chip surface, and the step of eluting the (specifically) bound phages.

The (bio)panning rounds can further comprise the step of clearing out aspecific membrane-binding phages.

Said clearing step can be performed, e.g. by covering the first quarter, the first half, the first three quarters of the flow cells of a sensor chip with plain liposomes (also referred to as blank liposomes), i.e. liposomes that do not contain the target membrane protein.

In particular, said clearing step can be performed, e.g. by covering the first one, first two, first three of the flow cells (or more depending on the total number of flow cells) of a sensor chip with plain liposomes.

And by running the solution(s) of the phage-displayed peptides through said first flow cell(s), before they are contacted with the target, aspecific (phospholipid) binding is minimized.

On the other flow cell(s) (the 4^{th} or the others if any, depending on the device), membrane protein containing liposomes (also referred to as proteoliposomes) are immobilized. And the solutions that have flowed over said first one, said first two and/or said first three of the flow cells (or more depending on the total number of flow cells), can be diffused over said other flow cell(s) bearing the membrane protein containing liposomes.

A method of the invention can further comprise the step of preparing the liposomes (blank liposomes and/or target membrane protein containing liposomes).

The liposomes can be prepared by means of an extruder and immobilized upon a sensor chip in accordance with the protocols disclosed in Cooper et al. 2000, analytical Biochemistry 277, 196-205.

Other methods commonly used in the field can also be carried out for preparing the liposomes.

Advantageously, in a method according to the invention, the liposomes (containing or not the target) are immobilized (or captured) upon the sensor chip by flowing over the flow cell(s) a solution of said liposomes at a slow flow rate, e.g. at rate of less than about 10 µl/min or less than about 5 µl/min, preferably less than about 4 µl/min or about 3 µl/min, and more preferably at flow rate of about 2 µl/min.

Preferably, a method of the invention further comprises a step of stabilizing the liposomes (containing or not the target), wherein a solution (a running buffer), which preferably does not affect the liposomes and the target protein(s), is flowed over the flow cell(s), preferably during at least 30 minutes, more preferably during at least 1 hour, in particular between about 30 minutes and about 12 hours, preferably between about 1 hour and about 5 hours. The running buffer can be for example an aqueous solution containing HEPES (4-(2-hydroxyethyl)-1-piperazineethanesulfonic acid) and sodium chloride, such as HBS-N^{®} buffer, can be also PBS (phosphate buffered saline), TBS (Tris buffered saline), etc.

The flow rate is preferably of less than about 20 µl/min, preferably of less than about 10 µl/min, and more preferably of about 5 µl/min.

Starting with a phage-expressed (naturally occurring or artificial) nucleic acid library (DNA, cDNA, RNA, double or single strand), (iterative) biopanning rounds are performed.

Advantageously, between each round, the positively selected phages are amplified (e.g. in E. coli) to reach an input titer value sufficient for ensuring that more than 1 copy, preferably more than 10 copies, more preferably more than 100 copies of each epitope/peptide is/are present statistically in the injected/perfused solution.

For example, starting with a titer value of 10⁷, the amplification should be performed to reach a titer value of at least 10⁹ so that at least 100 copies of each epitopes/peptides are (statistically) present in the injected solution, for the next round.

There is a relationship between such particle concentrations and the total volume of phage-containing buffer to be used for the biopanning round

Advantageously, in particular after each biopanning round, chloroform can be added to the eluted fraction(s) and/or to the solution(s) to be injected in order to prevent bacterial contamination and/or to clear said fraction(s)/solution(s).

Indeed, small amounts of chloroform (e.g. between about 5 vol. or wt.% and about 20%, in particular about 10%, preferably about 15%) induce most debris (for example the lysed cells) present in the fraction(s)/solution(s) to fall in the chloroform phase, and prevent the air bubbles formation that may break/interrupt the flow into the SPR device.

Air bubbles (or break-ups of the fluid column) are one of the most recurrent problems occurring during the selection rounds within a SPR device. And the recommended extra cleaning or rinsing steps are of no help to get rid of these bubbles generated by different phenomena such as the degassing of the phage solution, the clogging of the tube because of the solution crowding and/or viscosity, or the cavitation phenomena.

The use of chloroform is preferred to prevent or reduce the bubbles (or break-ups) formation, but replacing the tubes by Teflon® tubes may also allow to reduce this bubbles (or break-ups) formation.

Polyethylene glycol (PEG) may also be used to purify, by precipitation, the solution(s)/fraction(s). For example, a solution of PEG 6000 at about 50% in a ratio PEG /phage solution of 1:6 (vol/vol).

In a method of the invention, the perfusing step, wherein the phage displayed peptides contact with the target embedded in liposomes captured on at least one of the flow cells of a SPR device, is preferably performed as slow as technically possible in order to ensure an optimal competition between the present phages for binding the target. Advantageously, the flow rate is less than about 10 µl/min, preferably less than about 5 µl/min, 4 µl/min or 3 µl/min, and more preferably less than about 2 µl/min, and is in particular of about 1 µl/min.

Advantageously, in a method of the invention the eluting step is fractionated.

Different fractions can be collected at different points of time, or in function of time (periodically) and/or the eluted phages can be recovered for each flow cell, or any of the flow cells, or after the solution has flowed over one of the flow cells, over two, three, or four of the flow cells (or more depending on the SPR device used).

For eluting the bound peptides in a method of the invention, a suitable collecting solution, which preferably has no detrimental effect on the liposomes and the target, is injected. The eluted fraction(s) is/are then collected, e.g. for analysis or for further biopanning rounds.

Advantageously, the collecting solution is a buffer such as TBS, PBS, HSB-N, etc.

In a method of the invention, the flow rate for eluting the phages may vary according to the needs of the experimenter. Advantageously, the flow rate for eluting the phages is less than about 100 µl/min, preferably less than about 30 µl/min, or less than about 10 µl/min.

Preferably, a determined amount of buffer is injected, preferably at a very slow rate, e.g. less than about 10 µl/min, preferably less than about 5 µl/min, or 4 µl/min, or 3 µl/min, and more preferably less than about 2 µl/min, in particular at a rate of about 1 µl/min, and the eluted fraction is recovered in full. This step can be repeated once, twice, three times or more, resulting respectively in two, three, four or more fractions, each fraction corresponding to phages (phage displayed peptides) separated on basis of their dissociation rate constants.

It is also possible to perform this fractionating step continuously by perfusing the buffer and collecting the fractions at certain points of time or in function of time (periodically).

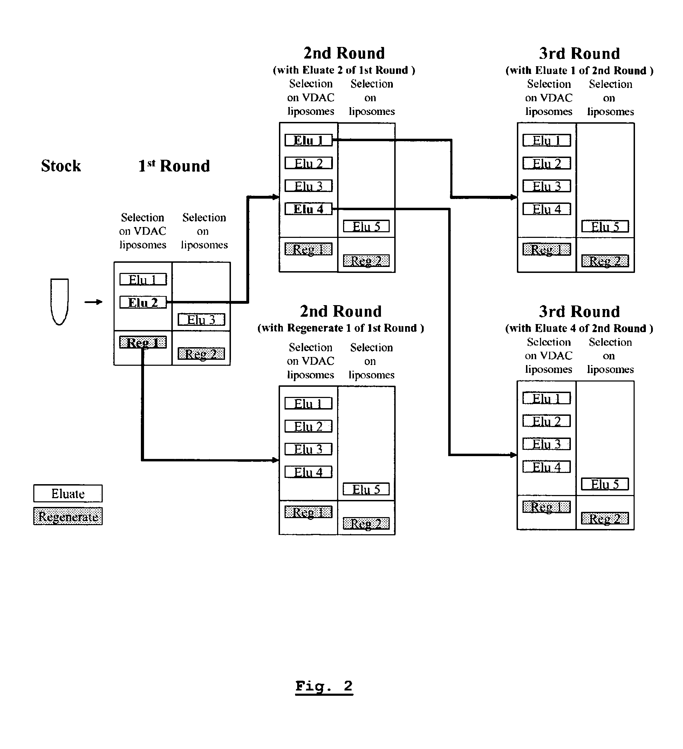

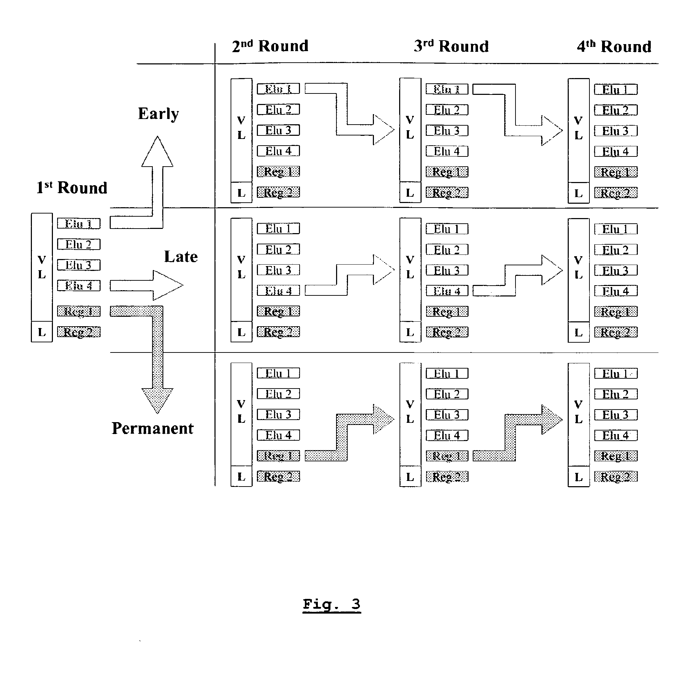

By fractionating the eluting step, it is possible to obtain eluted fractions containing phage displayed peptides differing in chemical affinities for the target. Therefore they can be separated and freely chosen to proceed further with a series of different experiments or analysis. In particular, they may serve in another biopanning round for enriching the selected phage population for the epitopes/peptides, displaying the level of desired affinity/avidity (see for example Figures 2 and 3).

Moreover, in a method according to the present invention, phages with (extremely) low dissociation rate constants can be collected, preferably after a fractionated eluting step, during a step referred to herein as the "(surface-)scraping procedure" or "scraping step". During this step, the liposomes are detached from the flow cell(s) surface and the remaining bound phages can be collected. Such collected phage displayed peptides correspond to nearly irreversible or even irreversible binders (at least in the conditions used in a method according to the invention).

For performing said scraping step, the sensor chip can be perfused with any suitable solution for releasing the liposomes. Advantageously, the solution used does not affect the phages still bound to the target. For example, a solution comprising less than about 1,5 wt.% SDS, preferably less than about 1 wt.% SDS and more preferably between about 1 wt.% and about 0,5 wt.% SDS, can be used, resulting in the liposomes to be detached from the sensor chip and the phages to be preserved. In order to avoid or minimize phages disruption, they should not stay in said solution more than about 5 minutes, preferably less than about 4 minutes and more preferably about 3 or 2 minutes.

Advantageously, said scraping step is performed prior to the next panning round for the chip surface to be regenerated.

Advantageously, said scraping step can be preceded by a (thorough) washing step with a solution that is not detrimental to the liposomes and target, e.g. by injecting during about 3 minutes at about 30 µl/min, a buffer such as PBS, TBS or HBS-N, etc.

In a method according to the invention, it is possible to independently perform different selection rounds for different phage fractions, resulting in the enrichment of the phage population with viral particles bearing peptides that can bind VDAC more or less tightly.

Many different strategies are possible (see for example Figures 2 and 3).

Advantageously, in a method of the invention, step (i), wherein said solution is any amplified eluted fraction(s) of previous step (iv), optionally step (ii), step (iii) and step (iv) are repeated twice, preferably three times, more preferably four times.

Nevertheless, they can also be repeated more than four times.

Advantageously, a method of the invention further comprises the step of identifying the peptide(s)/epitope(s) contained in at least one the eluted fractions of interest.

Advantageously, a method of the invention further comprises, before or after the step of identifying the peptides in at least one of the eluted fractions, the step of measuring the affinity of said peptides to the liposomes (proteoliposomes or blank liposomes) in the same SPR device.

Measuring the affinities of a mixture of peptides allows to control or to ascertain the selective enrichment of phages populations.

In a method according to the invention, the target protein embedded in the liposomes may be associated with another molecule (e.g. an integral, peripheral or anchored membrane protein known to interact with the primary target membrane protein), allowing the screening for ligands of higher order i.e. recognizing the complex formed by said target protein and said other molecule.

With a method of the invention, the competition problem encountered because of the plastic wells of Elisa tests, is strongly reduced and even eliminated.

Moreover, a method of the invention combines a successful reconstitution of membrane proteins within a lipid environment and a very stable solid-phase able to successfully perform the biopanning round (washing-out of poor binders).

Another advantage of a method according to the invention is the possibility of fractionating the output phages of the panning round according to the needs, in particular actually selecting ligands with desired affinities/avidities.

The selection performed according to a method of the invention could not have been achieved from panning usually performed under prior art conditions, e.g. in test-tube or in any set up favoring steady state binding.

In test-tube, the binding molecules are allowed to reach steady-state kinetics conditions, whereas by design, in a method according to the invention, the analyte molecules are submitted to a competition between diffusion and perfusion processes.

Indeed one advantage of the SPR device lies with its microfluidics: the epitope-bearing viruses are very slowly perfused (e.g. 1µl/min) in the small volume of a reaction chamber (e.g. 0.06 µl) e.g. for 100 min. These settings (or conditions) have various consequences for ligand selection.

In these conditions, the target receptors compete for binding during the association phase. Moreover, epitope re-binding is likely to occur during the dissociation phase.

And as a longer time is needed to reach a given binding level, multivalent binders - needing more time to bind - are favored.

In other words, in a method of the invention, by (time) fractionating under a continuous (buffer) flow the phage populations, a competition between the diffusion and the perfusion processes is generated, providing MTL conditions.

Because of these MTL conditions, the ligands availability is limited with respect to the target. The competition between ligands is thus higher, allowing selection of ligands with higher affinity. Moreover, the re-binding is favored; and in particular multivalent-ligands selection can be favored.

In a preferred embodiment, the target protein to be used in a method according to the invention is a mitochondrial outer membrane channel: a voltage dependent anion (selective) channel (VDAC) -purified 20 years ago and cloned 15 years ago-, which lies at the crossroad of several important cellular functions.

VDAC protein is a mitochondrial membrane channel found in every eukaryotic cell studied so far from all kingdoms of life. The VDAC channel proteins belong to a multigene family. With the notable exception of Saccharomyces cerev. (2 genes only) and Neurospora cr. (1 gene only), between 3 and 5 vdac genes have been found in every species where it was looked for.

As used herein, the term "VDAC protein(s)" refers to the different VDAC isoforms (i.e. from different genes or from different origins), except if the context dictates otherwise.

In mice the disruption of some of the genes has led either to the identification of neuromuscular syndromes or to asthenozoospermia.

In plants, VDAC has been reported to be involved in tissular differentiation.

In humans 3 genes have been found to date on 3 different chromosomes. In patients low levels of VDAC expression have been reported in encephalomyopathic children. And the expression level of VDAC can be used for diagnosing encephalitis or encephalopathy (WO 2004/077055).

VDAC is a voltage and a ligand-gated channel which, as a regulated passageway through the outer membrane, controls aerobic metabolism.

It is now admitted that it is involved in the control of aerobic metabolism as it forms the main protein pathway for metabolite diffusion across the mitochondrial outer membrane, and serves also as an anchoring and/or activating point for enzymes like hexokinase, glucokinase and glycerol kinase. It can thus regulate both the outer membrane permeability to the anionic metabolites, and the activity of some of the key metabolic enzymes generating those metabolites in the cytosol or in the mitochondria.

The channel appears also to be an important control point of apoptotic cascades via numerous protein-protein interactions.

Moreover, it is presumably involved in other mitochondrial functions via its associations with the peripheral benzodiazepine receptor, and via its associations with adenine nucleotide carrier.

Via numerous protein-protein interactions with members of the Bcl-2 family, the VDAC channel can integrate various cellular signals during the apoptotic processes.

Together with the adenine nucleotide translocator (ANT), some -but not all- of the mitochondrial VDAC proteins form one type of contact sites between the two mitochondrial membranes, such sites can presumably form the permeability transition pore (PTP) by binding cyclophilin D.

Such complex interactions highlight the various yet-to-be-understood links between apoptosis and metabolism.

Further, VDAC is also known to interact with cytoskeletal elements like MAP2, gelsolin, and G-actin and may thus partake in mitochondrial or cellular motility.

It interacts with other enzymes like the Kringle 5 domain of human plasminogen, endothelial nitric oxide synthase, and with itself.

Recent evidences also highlight a potential role in the nervous system function as a receptor for neurosteroids in close association with the GABA A receptor channel.

Moreover, its lack of function or its decreased expression leads to various pathologies, including -but not limited to- encephalomyopathic syndromes, asthenozoospermia, cognitive deficits, etc.

VDAC is thus a multifunctional protein located in the mitochondrial outer membrane where it can serve either as a channel, as a receptor or as a regulatory molecule.

All these multiple and complex protein-protein interactions -most of them indirectly obtained - warrant a systematic search of the VDAC interacting map of proteins or interactome.

Ligands that could thus selectively interfere directly with the channel - i.e. keep it either open or closed - or indirectly with some of its protein interacting sites would thus be important pharmacological tools as well as potential valuable medications.

Unfortunately, a lot of the information pertaining to the many reported VDAC-protein interactions relies on indirect evidences or on knock-out experiments that do not always yield univocal interpretations.

Indeed, depending on the experimental - i.e. reductionist - conditions, some interactions may be favored against others or may even be due to altered protein folds depending on the test conditions.

A method according to the present invention allows the selection and identification of VDAC-binding polypeptides that show different degrees of affinity or avidity (small, high or very high) to VDAC, which is virtually in its native form.

A method according to the invention combines:
- exploring a set of phage-displayed (naturally occurring or artificial) peptides,
- using the native fold of VDAC, which is achieved by its functional incorporation into liposomes,
- using a surface plasmon detector device (SPR).

In a method of the invention, a naturally occurring or artificial nucleic acids library can be screened for VDAC ligands with the phage display methodology optimized to target VDAC, possibly in its different isoforms, reconstituted into a membrane environment, using the SPR technology.

To find the various peptides binding to VDAC in its native form, VDAC protein can be reconstructed into liposomes, which are then fixed on a chip, e.g. a Biacore® 2000 sensor chip, in particular a L1 chip.

A library can be screened with the Phage Display technique within the SPR biosensor device itself.

According to the invention, a method for screening VDAC ligands, as a specific case of the method of claim 13, comprises the steps of:
i. preparing a solution containing a library of phage-displayed peptides,
ii. immobilizing the VDAC proteins incorporated into liposomes, preferably into large unilamellar vesicles (LUV), upon at least one of the flow cells of a sensor chip in a plasmon detector (SPR) device,
iii. flowing said prepared solution over said flow cell(s) of said sensor chip,
iv. eluting the phage displayed peptides, and
v. optionally, repeating at least once:
   - step (i), wherein said solution is any amplified eluted fraction(s) of previous step (iv),
   - optionally step (ii),
   - step (iii), and
   - step (iv).

In the context of the present invention, the steps (iii) and (iv) are performed inside the chamber of a SPR biosensor device, e.g. Biacore® 2000 device.

The (bio)panning rounds can further comprise the step of clearing out aspecific VDAC-binding phages.

Said clearing step can be performed, e.g. by covering the first one, first two, first three of the flow cells (or more depending on the total number of flow cells) of a sensor chip with plain liposomes. By running the solution(s) of the phage-displayed peptides through said first flow cell(s), before they are contacted with the VDAC proteins, aspecific (phospholipid) binding is minimized.

On the other flow cell(s) (the 4^{th} or the others if any, depending on the device used), VDAC containing liposomes are immobilized. And the solutions that have flowed over said first one, said first two and/or said first three of the flow cells (or more depending on the total number of flow cell), can be diffused over said other flow cell(s) bearing the VDAC containing liposomes.

A method of the invention can further comprise the step of preparing the liposomes (blank liposomes and/or VDAC containing liposomes) .

The liposomes can be prepared by means of an extruder and immobilized upon a sensor chip in accordance with the protocols disclosed in Cooper et al. 2000, analytical Biochemistry 277, 196-205 (see example 2).

Other methods commonly used in the field can also be carried out for preparing the liposomes.

Advantageously, in a method according to the invention, the liposomes (containing or not VDAC proteins) are immobilized (or captured) upon the sensor chip by flowing over the flow cell(s) a solution of said liposomes at a slow flow rate, e.g. at rate of less than about 5 µl/min, preferably less than about 4 µl/min or about 3 µl/min, and more preferably at a flow rate of about 2 µl/min.

Preferably, a method of the invention further comprises a step of stabilizing the liposomes (containing or not VDAC proteins), wherein a solution (in particular a buffer such as PBS, TBS, HBS-N, etc.), which has preferably no detrimental effect on the liposomes, is flowed over the flow cell(s), preferably during at least 30 minutes, more preferably during at least 1 hour, in particular between about 30 minutes and about 12 hours, preferably between about 1 hour and about 5 hours.

The flow rate is preferably of less than about 20 µl/min, preferably of less than about 10 µl/min, and more preferably of about 5 µl/min.

Starting with a phage-expressed (naturally occurring or artificial) nucleic acid library, (iterative) biopanning rounds are performed.

Advantageously, between each round, the positively selected phages are amplified (e.g. in E. coli) to reach an input titer value sufficient for ensuring that more than 1 copy, preferably more than 10 copies, more preferably more than 100 copies of each epitope/peptide is/are present statistically in the injected/perfused solution.

For example, starting with a titer value of 10⁷, the amplification should be performed to reach a titer value of at least 10⁹ so that at least 100 copies of each epitopes/peptides are present in the injected solution, for the next round.

There is a relationship between such particle concentrations and the total volume of phage-containing buffer to be used for the biopanning round.

Advantageously, in particular after each biopanning round, chloroform can be added to the eluted fraction(s) and/or to the solution(s) to be injected in order to prevent bacterial contamination and/or to clear said fraction(s)/solution(s).

Indeed, small amounts of chloroform (e.g. between about 5 vol. or wt.% and about 20%, preferably between about 10% and about 20%, in particular about 10%, preferably about 15%) induce most debris (for example the lysed cells) present in the fraction(s)/solution(s) to fall in the chloroform phase, and prevent the air bubbles formation that may break/interrupt the flow into the SPR device.

The tubes may also be replaced by Teflon® tubes for reducing the bubbles (or break-ups) formation.

Polyethylene glycol (PEG) may also be used to purify, by precipitation, the solution(s)/fraction(s).

In a method of the invention, the perfusing step, wherein the phage displayed peptides contact with VDAC proteins embedded in liposomes captured on at least one of the flow cells of a SPR device, is preferably performed as slow as technically possible in order to ensure an optimal competition between the present phages for binding the target. Advantageously, the flow rate is less than about 10 µl/min, preferably less than about 5 µl/min, 4 µl/min or 3 µl/min, and more preferably less than about 2 µl/min, and is in particular of about 1 µl/min.

For eluting the bound peptides in a method of the invention, a suitable solution is injected. The eluted fraction is then collected, e.g. for further biopanning round or for analysis.

For example, the solution is buffer such as PBS, TBS, HBS-N, etc.

Advantageously, in a method of the invention the eluting step is fractionated.

Different fractions can thus be collected at different points of time, or in function of time (periodically) and/or the eluted phages can be recovered for each flow cell, or any of the flow cells, or after the solution has flowed over one of the flow cells, over two, three, or four of the flow cells (or more depending on the SPR device used).

Preferably, a determined amount of buffer is injected, preferably at a slow rate, e.g. less than about 10 µl/min, preferably less than about 5 µl/min, or 4 µl/min, or 3 µl/min, and more preferably less than about 2 µl/min, in particular at a rate of about 1 µl/min, and the eluted fraction is recovered in full. This step can be repeated once, twice, three times or more, resulting respectively in two, three, four or more fractions, each fraction corresponding to phages (phage displayed peptides) separated on basis of their dissociation rate constants.

Advantageously, this fractionating step is continuous by perfusing the buffer at a flow rate of e.g. less than about 10 µl/min, preferably less than about 5 µl/min, and recovering an eluted fraction at certain points of time or in function of time.

By fractionating the eluting step, it is possible to obtain eluted fractions containing phage displayed peptides differing in chemical affinities/avidities for the VDAC protein. Therefore they can be separated and freely chosen to proceed further with a series of different experiments or analysis. In particular, they may serve in another biopanning round for enriching the selected phage population for the epitopes/peptides, displaying the level of desired affinity/avidity.

In a method of the invention, the flow rate for eluting the phages may vary according to the needs of the experimenter. Advantageously, the flow rate for eluting the phages is less than about 100 µl/min, preferably less than about 30 µl/min, or less than about 10 µl/min.

Moreover, in a method according to the present invention, phages with extremely low dissociation rate constants (at least within the conditions used in a method of the invention) can be collected, preferably after a fractionated eluting step, during a scraping step. During this step, the VDAC containing liposomes are detached from the flow cell(s) surface and the remaining bound phages can be collected. Such collected phage displayed peptides correspond to nearly irreversible or even irreversible binders.

For performing said scraping step, the sensor chip can be perfused with any suitable solution for releasing the liposomes. Advantageously, the solution used does not affect the phages still bound to the VDAC proteins. For example, a solution comprising less than about 1,5 wt. % SDS and more than about 0,5 wt.% can be used, preferably about 1 wt. % SDS, resulting in the liposomes to be detached from the sensor chip and the phages to be preserved.

Advantageously, a method of the invention further comprises the step of identifying the peptide(s)/epitope(s) contained in the elution fractions of interest.

In a method according to the invention, the VDAC proteins embedded in the liposomes may be associated with another molecule (e.g. an integral, peripheral or anchored membrane protein known to interact with VDAC proteins), allowing the screening for ligands of higher order i.e. recognizing the complex formed by said VDAC proteins and said other molecule.

After at least 1 but preferably 2 or 3 and more preferably 4 biopanning rounds against purified Yeast VDAC proteoliposomes immobilized onto the surface of a SPR chip, positive selection against control liposomes is obtained.

From the positive output phages, one to several hundreds of individual clones can be amplified, their affinity to VDAC can be confirmed and their effect on VDAC function can be investigated.

The DNA insert of each positive clone can be sequenced.

For example, carrying out a method according to the invention for screening and selecting VDAC ligands from a human liver cDNA library, using the SPR technology, the DNA insert of one of the positive clones was sequenced and yielded a 224 bp sequence that matched a portion of the subunit I of human cytochrome c oxidase (COX), an enzyme located in the mitochondrial inner membrane.

The functional implications of this interaction were evidenced by assaying the effect of VDAC on the oxidation of cytochrome c by the pure bovine holoenzyme.

Further, using a liposome-based VDAC permeability assay, the interacting COX segment was found to markedly decrease the permeability to polyethylene glycol (PEG 800) a VDAC permeable molecule.

These results which are proof that the COX epitope binding to VDAC is likely relevant for cell regulation provide also strong evidence for the presence of a novel type of contact sites between both mitochondrial membranes.

The further exploration of the physiological relevance of such COX-VDAC interaction may provide a mechanistic explanation for some reported cognitive deficits observed in VDAC defective transgenic mice linked to reduced cytochrome C oxidase activity (Scaglia N. et al. Mitochondrial porin deficient mice: A murine model of Mendelian respiratory chain defects and encephalopathy. 17-9-2002. Workshop: Systems Biology Approaches to Health Care: Mitochondrial Proteomics. 17-9-2002.ref. of publication(s); and Wu S. et al. (1999) Biochemica et Biophysica Acta 1452, 68-78).

As the orientation of the VDAC channel ought to be random following liposome incorporation, both cytosolic and mitochondrial epitopes/peptides are likely to be encountered during the screening process.

In a method of the invention, in the perfusing step, flowing the batch of input phages over the control lipid surfaces prior to the VDAC liposomes minimizes the selection for aspecific binders.

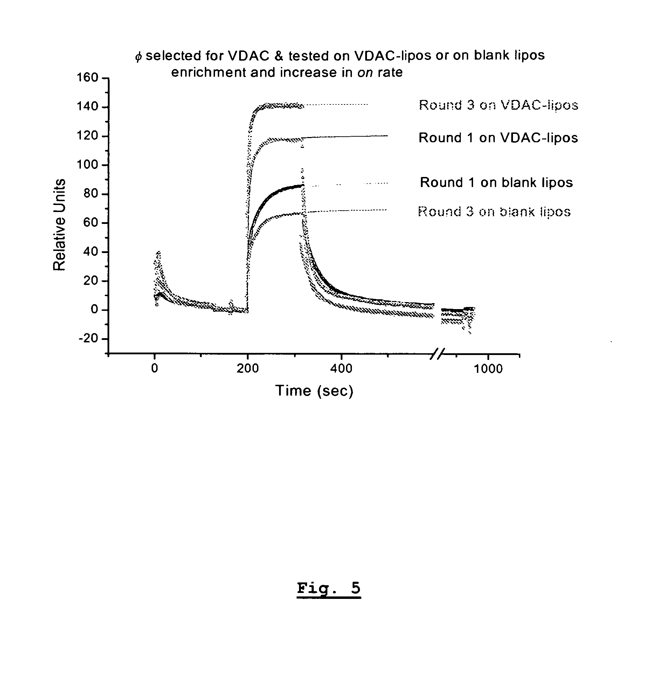

As shown in Figure 5 with the affinity testing of the bulk output phage population, flowing the phage solution over at least one of the flow cells covered with plain liposomes does minimize the selection for aspecific binders.

In a method according to the invention, it is possible to independently perform different selection rounds for different phage fractions, resulting in the enrichment of the phage population with viral particles bearing peptides that can bind VDAC more or less tightly.

From the positively selected phages, an individual clone could be identified that carried a rather large portion of the cytochrome c oxidase subunit I (COX subunit I) as a fusion protein on its envelope.

Located within the mitochondrial inner membrane, COX is the terminal enzyme of the electron transport chain. The functions of this 205 kDa protein, i.e. Cyt c oxidation, electron transfer to oxygen and proton pumping are mainly ascribed to its subunits I and II, and three active redox metal centers are bound to its subunit I.

The eukaryotic COX consists of a total of 13 subunits, three of which -subunit I, II and III - are encoded in the mitochondrial DNA. These three subunits carry out the catalytic functions of COX and are highly conserved between pro- and eukarya.

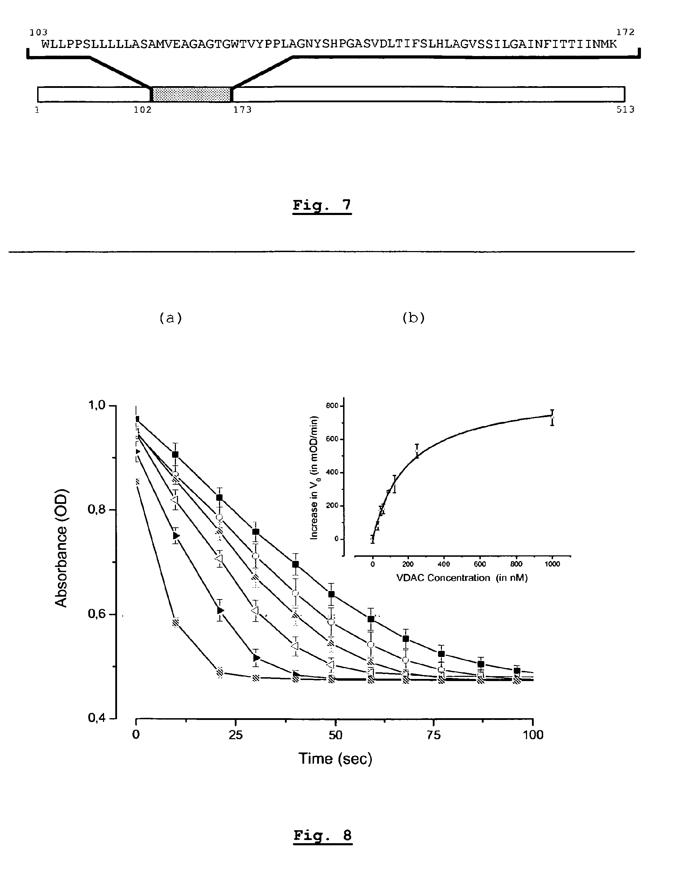

After analyzing the corresponding phage-displayed peptide, it could be inferred that the interacting COX I region is located between its residues 103 to 173 (see figure 7).

Also disclosed is isolated nucleic acid molecule comprising or consisting of :
- a nucleotide sequence of SEQ ID NO 1, its complementary form or RNA form,
- a nucleotide sequence having at least 80%, advantageously at least 85%, more advantageously at least 90%, preferably at least 95%, more preferably at least 96%, 97%, 98% or 99% homology or identity with SEQ ID NO 1, or with its complementary form or RNA form,
- any fragment thereof of at least 20 nucleotides, more preferably of at least 25, 30, 35, 40 or 50 nucleotides, wherein said fragment encodes a fragment of a polypeptide of SEQ ID NO 2, or a fragment of a polypeptide having at least 90%, preferably at least 95%, more preferably at least 96%, 97%, 98% or 99% homology or identity with SEQ ID NO 2.

Also disclosed is an isolated amino acids molecule comprising or consisting of SEQ ID NO 2 or any sequence that shows a homology or identity of at least 90% with SEQ ID NO 2, preferably of at least 95%, 96%, 97%, 98% or 99% homology or identity with SEQ ID NO 2, or any fragment thereof of at least 10, 15 or 20 amino acids.

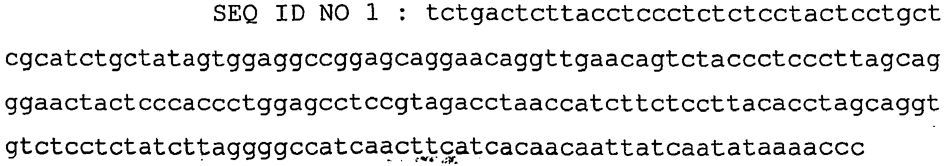

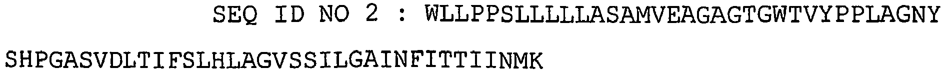

Although the main binding site for Cyt c lies in subunit II, several amino-acids from the subunit I located at position 50 and 221 seem involved in the molecular recognition between the enzyme and the electron carrier.

As far as the VDAC domain interacting with COX is concerned, it is probable that it lies within one of the few short extra-membrane loops that the VDAC protein exhibits.

Direct binding between the two (pure) proteins can be obtained and measured using surface plasmon resonance.

To further ascertain the eventual relevance of the VDAC-COX binding i.e. to reveal a direct functional interaction between these two proteins, an in vitro assay based on the enzymatic function of COX was performed.

A 'chimeric' COX assay using both bovine gene products (holoenzyme, Cyt c) and yeast (VDAC) gave enough signal-to-noise ratio to ascertain sizeable variations in enzyme activity.

A 3 to 4-fold acceleration in the rate of Cyt c oxidation could be measured using nanomolar concentrations of VDAC.

It must be pointed out that, in the assay, the detergent concentration was kept constant at all the VDAC doses tested (including in VDAC absence).

From the assay performed in bulk phase conditions, an apparent K_{D} value of 200 nM of VDAC for COX could be derived. The real value in the cell should be orders of magnitude lower as both these proteins have their free diffusion limited in the membrane bi-dimensional spaces and as the two mitochondrial membranes are very close to each other.

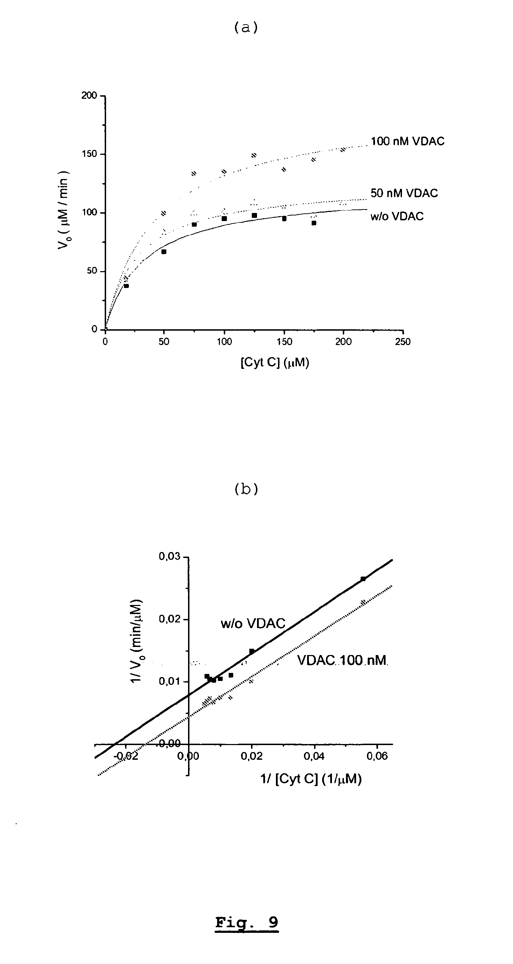

Taken together the enzymatic data are consistent with an uncompetitive activation process (see figure 9). Such processes where both Vₘₐₓ and K_{M} are increased are already described. COX rejoins thus other enzymes that can be activated upon binding to VDAC : hexokinase, mitochondrial creatine kinase, plasminogen, eNOS, to cite a few.

This fact shows that Cyt C and VDAC do not share the same binding site on COX. COX can thus be used in a method for detecting and/or quantifying VDAC. Indeed, the increased reaction rate of cytochrome c oxidase induced by a preparation to be tested for VDAC presence and/or quantity can be used as a signal directly or indirectly to detect and/or quantify VDAC binding.

It is also worth noting that the functional interactions occur in the absence of the ANT or any other PTP-related component.

Another functional evidence was obtained by measuring the effects of the COX epitope on the VDAC permeability after reconstitution into large unilamellar vesicles.

Depending on the ionic and/or colloidal conditions, the volume of these proteoliposomes may vary and these variations in volume can be acquired as variations in light scattering measurable by a spectrophotometer at 400 nm.

As the light scattering signal contains also information on the surface chemistry of the proteoliposomes, such experiments should be perfectly calibrated i.e. all variables should be identical but the nature of the tested ligand.

Taking advantage of the differential VDAC permeability displayed by identical concentrations of PEG 800 and PEG 1500, VDAC closure could be reliably measured by the slope of the re-swelling of the proteoliposomes consecutive to the addition of a fixed concentration of PEG800, a VDAC-permeable osmoticant.

Upon addition of the osmoticant molecule, VDAC liposomes first rapidly shrink due the osmotic shock then re-swell to a steady-state value only if the osmoticant is permeable through the VDAC channel.

Liposomes devoid of any channel protein just shrink to a certain steady-state value.

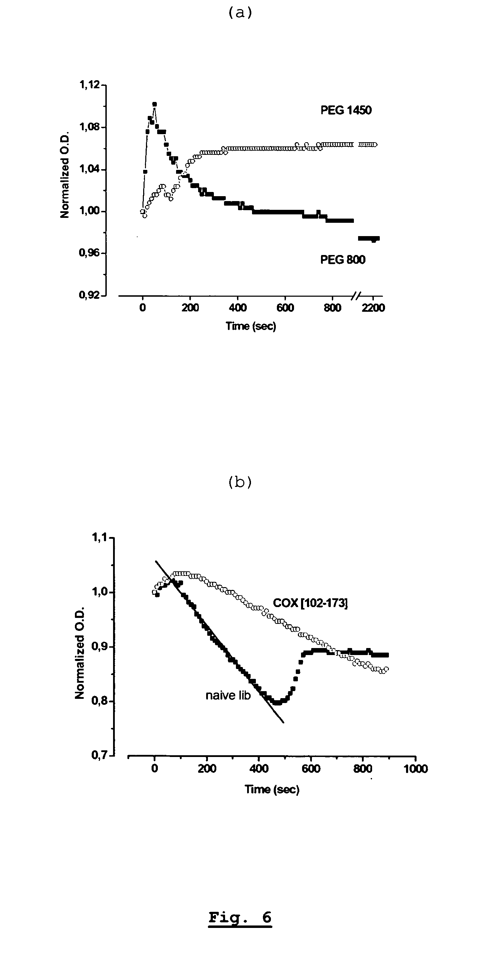

When the channel is closed or when PEG1500 is used, there is barely any re-swelling following the osmotic challenge (see figure 6).

As this assay is sensitive to dilution effects, identical volumes of phage-containing solutions were always used for the experiments.

Also disclosed is a new VDAC permeability test using unilamellar vesicles (SUV, small unilamellar vesicles, with a diameter of less than 50 nm, or LUV, large unilamellar vesicles, preferably with a diameter of more than 50 nm, more preferably about 100 nm) osmotically challenged by PEG 800.

This VDAC permeability test offers a few advantages over other liposome-based VDAC permeation testing. As it is based on unilamellar vesicles, and not on multilamellar or multiwalled liposomes, the signals are obtained faster : few seconds to few minutes, in particular less than 10 minutes, instead of tens of minutes typically. This gives a low-level scattering signal but the proteoliposomes concentrations can be increased to easily circumvent that limitation.

The phage concentrations used in these experiments -as determined by titration- ranged between 1,5 and 0,15 nM. Such variations can be considered trivial as far as permeability effects are concerned.

It can be clearly observed that the phage bearing the COX sequence but not the naive phage particles decrease the slope of re-swelling. This would be consistent with the COX protein favoring VDAC closure.

Recent reports linking VDAC to the export/release of free radicals from the mitochondrial spaces hint at a potential role for VDAC in the control of either the free radical potential and/or some redox potential in the intermembrane space.

The situation is likely complex given that VDAC can selectively bind to endothelial Nitric Oxide Synthase (eNOS) and up-regulate its NO production and that NO itself is reported to compete with O₂ on COX and, therefore NO could regulate the oxidase activity.

Nevertheless these new data strongly suggest the existence of a novel type of contact site between the two mitochondrial membranes.

Regardless of the intricate free radical chemistry involved here, these data already provide a straightforward molecular mechanism for the cognitive deficits measured in transgene mice lacking MVDAC1 or MVDAC2 and showing a concomitant marked decrease in COX enzymatic activity.

### Description of the figures and drawings

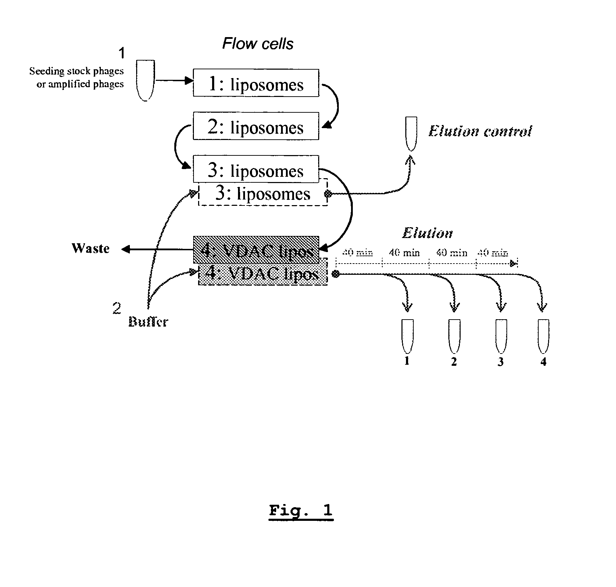

Figure 1 shows an example of SPR-based experimental flow chart performed during each panning round. The flow cells (FC) are prepared with liposomes (FC #1 to 3) or proteoliposomes (FC#4) as their fixed phase.

Firstly, the naive (unselected) phage stocks or the re-amplified (after the n^{th} panning round) output phage population are perfused in the SPR device, allowed to pass sequentially over the first 3 cells, then to compete for target binding (VDAC) in the last cell, the overflow of the perfusing solution being discarded (waste).

Then, one (or more) of the target-free cell (containing the aspecific lipophilic or phospholipid -specific phages) and the test cell are perfused with a collecting solution to collect the bound phages. Several output phage population are then obtained : a "control" phage population and 4 target-selected phages populations obtained by fractionation as a function of time during the test cell elution.

After a scraping step, the various phage populations of interest are re-amplified on their host cells and purified and are possibly used for the next panning round.

Figure 2 shows one the many possible paths that can be used during the iterative selection process.

With the fractionating step, the Darwinian selective pressure can be put where appropriate. For example, on this figure, after the first round, a late VDAC-selected fraction is further pushed via selection for the second round where the output is then split in early and late and these two output phage populations can be independently further selected during a third panning round.

In parallel, during the same series of experiments, the "irreversibly bound" fraction collected after the first panning round and after the scraping step, is also independently re-amplified, further perfused and then eluted with a fractionating step.

Figure 3 shows an elution scheme for the selection of VDAC binding phages with different affinities.

In the first panning round four different fractions are collected from the flow cell with immobilized VDAC proteoliposomes at four consecutive elution times (Elu 1,2,3,4).

After the elution, the chip is further scraped (detergent treatment) and near irreversible binders are collected in the regenerate (Reg 1).

As a control the latter deterging procedure is also performed on the flow cell with immobilized blank liposomes (Reg 2).

All of these collected samples are then used as seeding stock for a next round of panning.

The scheme shows within the horizontal panels, the iterative selection procedure for either low affinity (early), or high affinity (late) or near irreversible (permanent) binders over four rounds of biopanning.

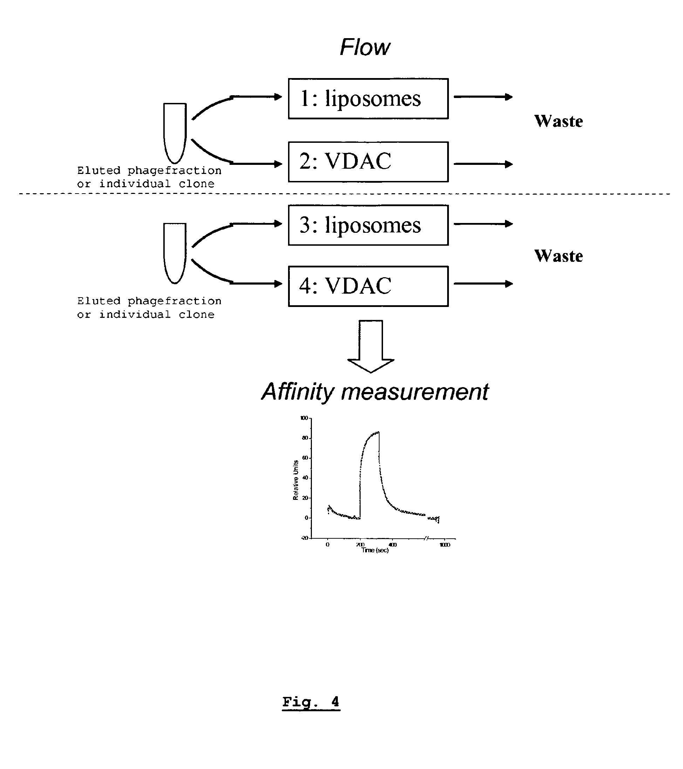

Figure 4 shows an affinity measurement as performed after each/a panning round.

In separate experiments, after each round of panning, the output phage populations can be tested for their selective enrichment in binding affinity for the target protein (VDAC) in a biosensor device.

The 4 flow cells are used (two prepared with blank liposomes, two prepared with proteoliposomes), every sample is assayed on the two matched (test vs. control) flow cells and SPR signals are compared after appropriate signal processing (see lowest part of the figure).

The same experimental set-up can be used to assay the binding characteristics of some individual phage clone.

In figure 5 are represented the affinities of the bulk phage populations for either plain liposomes or VDAC-proteoliposomes after 1 or 3 rounds of selection against VDAC-proteoliposomes. The affinity measurements are performed by a SPR device.

The VDAC-proteoliposomes signal increases between round 1 and 3 whereas the plain liposomes signal decreases.

The data show hence an increased specificity for VDAC-proteoliposomes and therefore a selective enrichment of the phage population for the target membrane protein (VDAC).

To be noted, the difference in on-rate of binding between the VDAC-lipos and the blank lipos curves.

Figure 6 shows that COX decreases VDAC permeability to PEG 800.

The volume changes of VDAC-containing proteoliposomes (measured as light scattering at 400 nm) are plotted as a function of time following the addition of a fixed volume (10 µl) of PEG 800 or PEG 1450 used as osmoticants at 10 mM.

On figure 6a, following an initial shrinkage due to the osmotic shock, the proteoliposomes re-swell only for the PEG 800, evidence that PEG 800 can re-equilibrate across the liposome membrane and is thus VDAC permeable (closed squares). This is not the case for the heavier colloid PEG 1450 in identical conditions (open circles).

On figure 6b, an identical experiment is performed using PEG 800 only. The swelling rate is different when the proteoliposomes - from the same batch - are pre-incubated and equilibrated with the initial naive phage library (closed squares) or the pure COX-presenting phage clone (open squares). The linear fit being proportional to the channel permeability, the difference in slope corresponds to a 2.3 fold decrease in VDAC permeability. This experiment was repeated with consistent results (for conditions see examples section).

Figure 7 represents COX I region expressed by the VDAC-interacting clone.

The VDAC-binding human COX I epitope is shown (shaded). The epitope spans residues 103 to 172. The residues 118 to 140 are reported facing the mitochondrial intermembrane space (from SWISSPROT P00396), the rest of the sequence corresponding to transmembrane segments.

Figure 8 shows that pure VDAC enhances 4-fold the oxidation of Cyt c by the COX holoenzyme.

Enzymatic oxidation of 109 µM of cytochrome c by 200 nM of bovine COX in presence of increasing yeast VDAC concentrations (1µM gray squares, 250 nM black triangles, 125 nM white open triangles, 60 nM grey triangles, 30 nM open circles, 0 M black squares).

The oxidation is followed as a decrease in absorbance spectra at 550 nm in a Biorad plate reader. The plates are shaken during readings (figure 8a).

Initial velocities (V₀), derived from the initial linear fits of the absorbance data are plotted as a function of VDAC concentration in inset (figure 8b).

Assuming a one-to-one interaction between VDAC and enzyme, the KD -derived from the simple hyperbolic fit- is 200 nM.

Assay conditions are 9.6 mM Tris-HCl pH 7, 635 µM HEPES, 116 mM KCl, 23 µM EDTA, detergent concentration (0.002% Dodecyl Maltoside, 0.023% Triton X-100) kept constant at all VDAC concentrations.

Figure 9 shows that VDAC is an uncompetitive activator of COX.

Are represented on figure 9a, initial velocities of the COX holoenzyme as a function of cytochrome c concentrations for 3 different VDAC levels -none (black squares), 50 nM (open triangles) and 100 nM (closed circles).

Assay conditions are identical to Figure 8, except for the Cyt c concentrations.

In the right panel (figure 9b), the same data are plotted as a Lineweaver-Burk plot (at 2 VDAC doses for clarity) evidencing that the interaction of Cyt c and VDAC for the COX enzyme is not competitive.

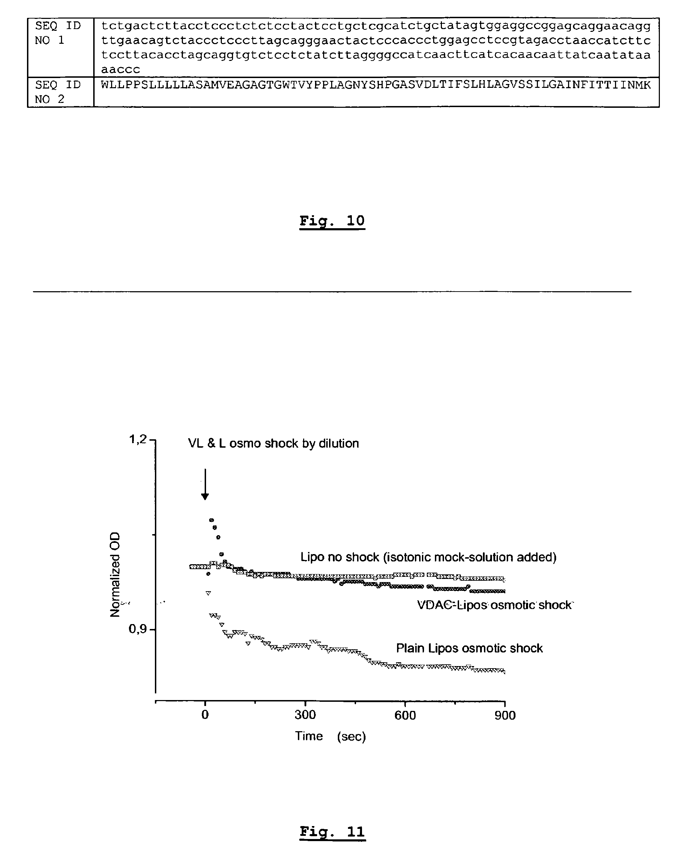

Figure 11 shows the normalized Optical Density (scattering at 400 nm) as a function of time for either plain liposomes, or VDAC proteoliposomes following an osmotic shock (at arrow) obtained by dilution of the buffer solution (initially KCl 2 M inside and outside the liposomes, after shock 1 M outside).

It can be observed that : (i) without any osmotic shock, i.e. addition of a volume of isotonic solution, the signal stays stable (gray squares), (ii) with the osmotic shock, i.e. external dilution, the liposomes rapidly swell to a new stable value reflecting the set osmotic gradient - this is measured by a decrease in OD- (gray triangles), (iii) with the protein successfully reincorporated into the liposomes, following the osmotic shock, after an initial signal disturbance, the liposomes do not swell noticeably (black losanges). This is due to the near-instantaneous osmotic gradient equilibration that the VDAC pore would allow.

### EXAMPLES

### Example 1: Reagents

All reagents were analytical grade.

Crystal grade Cytochrome c oxidase was kindly provided by Dr. Verkhovsky, Helsinki, Finland. VDAC from Yeast was purified to near homogeneity according to the method of De Pinto et al (Biochim.Biophys.Acta 905, 499-502 (1987) and J.Bioenerg.Biomembr. 21, 417-425 (1989)).

Phospholipids were L-a-phosphatidyl choline Type II-S from soybean (Sigma-Aldrich, Belgium). Running buffer HBS-N (0.15 M NaCl, 10 mM HEPES, pH 7.4), filtered and degassed (Biacore).

### Example 2: Liposome preparation.

Phospholipids (1.52 mg) with 20% cholesterol (0.3 mg) were dissolved in 1 ml hexane in a 10 ml round bottom flask. A thin lipid film was deposited by evaporation of hexane under a filtered N₂-stream (0.22 µm filter).

One ml of experimental buffer (1 M KCl, 5 mM CaCl₂, 10 mM HEPES pH 7.2, filtered through 0.22 µm filters) was added to obtain a 20 mM suspension, and multi-lamellar vesicles (MLV) were formed by extensive vortexing.

The lipid suspension was then submitted to 4 freeze-thaw cycles and, to a 10 sec. sonication period to yield large unilamellar vesicles (LUV).

Finally the size of these vesicles was homogenized by pushing the suspension 25-30 times through a 100-nm polycarbonate filter in a mini extruder (Avanti® Polar Lipids, inc).

### Example 3: VDAC-liposomes

VDAC-liposomes were obtained by diluting (1:3, vol/vol) of the previous liposome (LUV) suspension with 50% experimental buffer (as above) and, by adding twice - separately- 50 µl of a purified VDAC-containing solution (1 mg/ml VDAC in 5 mM Tris pH7, 0.5 mM EDTA, 2.5 mM KH₂P0₄, 25 mM KCl / 1 % Triton) to 400 µl liposomes, waiting each time 10 min for VDAC to insert.

The VDAC proteoliposomes were then diluted (1:3, vol/vol) in an appropriate buffer (30 mM KCl, 10 mM HEPES, pH 7.4 filtered through 0.22 µm filters) to obtain a final VDAC-LUV suspension with a very low final triton concentration (0.05%), a physiological osmolarity (300 mOsm) and a lipid concentration of 1 mM.

Blank liposomes (without any protein) were made and diluted in the same fashion.

### Example 4: Liposome immobilisation on a L1 Sensor Chip

Vesicles were captured on the L1 Sensor Chip as previous described.

Shortly, the surface of a L1 Sensor Chip was cleaned by a 2-min injection of 20 mM Chaps at a flow rate of 20µl/min, followed by the 'extraclean' rinsing routine. HBS-N was used as running buffer after filtration and degassing.

Liposomes (80 µl, 1mM of lipids) were then immediately injected at a flow rate of 2µl/min.

The fixed lipid layer was then washed at a flow rate of 100 µl/min with sodium hydroxide (10 mM, 50 µl).

In the case of VDAC-liposomes, 120 µl of 1 mM proteoliposome solution at the same flow rate were used.

Following a two hour long control period-(running buffer at 5 µl/min) to ascertain the stability of the fixed phase in the biosensor chamber, the degree of coverage of the chip's surface was determined by the ratios of the background reading signals (in relative resonance units, RU) in the presence or the absence of liposomes.

The un-covered chip-surface was then blocked with bovine serum albumin (BSA) (0.1 mg/ml, injection of 25 µl at 5 µl/min).

### Example 5: Biopanning of the phage library against VDAC-liposomes

The pre-made T7-SelectTM Liver library (Novagen, Madison, WI) was used for selecting the phages displaying expressed epitopes binding to VDAC. This cDNA library expresses its inserts fused to the C-terminus of the T7 gene10B major capsid protein, with an average of 10 copies displayed per virion, and inserts range from 300 bp to 3000 bp.

The biopanning rounds were performed in a Biacore 2000 instrument (Biacore AB, Uppsala, Sweden) using a L1 Sensor chip and HBS-N (above) as running buffer.

### Example 6: Phage selection.

To select for phages that bind to VDAC, the initial library was amplified and injected at very low flow rate (1 µl/min) over a chip covered with VDAC-liposomes (~ 5000 RU level).

Such low flow rate allows a better competition between the viral particles for the binding sites.

To filter out the phages bearing plain lipophilic epitopes or epitopes targeted to BSA - i.e. to increase the specific signal to noise ratio -, the phage solution is first flushed at the same flow rate over 3 flow cells covered with blank liposomes (covered at ~ 7000 RU level) and blocked with BSA before being allowed to reach the 4th flow cell covered with VDAC-liposomes.

A titer of 10⁹ phages/100 µl was used to cover the whole range of the library sequence space (10⁸ variants/ml according to the manufacturer's manual).

Such panning rounds were repeated 3 times.

### Example 7: Phage elution.

Following the competition and presumably the binding phase, the reaction chamber was briefly washed with buffer (5 min at 1 µl/min) to remove any unbound or poorly bound material.

To recover VDAC-binding phages based on different dissociation rates, collection over sequential time intervals were performed from flow cell #4.

A fixed amount of buffer was injected at 1 µl/min and recovered in full every 40 min, this was performed 4 times yielding thus 4 fractions separating the phages based on their dissociation rate constants.

As a control the same procedure was used over flow cell 3 (liposome binding phages).

As prior to the next panning round the chip surface was regenerated, the phages with extremely low dissociation rate constants (near 'irreversible' binders) were collected during the regeneration procedure.

Therefore the sensor chip was first washed thoroughly by injection of buffer at high flow rate (90 µl at 30 µl/min), followed by an injection of 1% Sodium Dodecyl Sulphate (SDS) (6 µl at 2µl/min) to remove phages and liposomes from the sensor surface.

T7 phages are resistant to a brief exposure (minutes) to 1% SDS and if directly diluted after recovery (SDS-samples are brought to a volume of 30 µl with HBS-N buffer), they can safely be titrated and propagated.

### Example 8: Phage amplification and titration.

Phage titers were determined by infection of 250 µl of BLT5615 cells, harvested in presence of carbenicillin (50 µg/ml), with 100 µl of a 10-fold dilution of the eluted phages.

The cell-phage samples were added to 3 ml H-top agar containing 4 mM IPTG and plated on LB agar supplemented with carbenicillin.

IPTG is required to induce the production of the 10A capsid protein of the T7 phages.

The plates were left overnight at room temperature; the number of plaque forming units (pfu) was counted for all samples.

To amplify the phages in between each selection round, 10 ml of BLT5615 cells (harvested at log phase in presence of carbenicillin and IPTG 1mM) were infected with 20 µl of phage eluate.

Incubation for 1,5 hour at 37 °C in a sterile chamber caused complete lyses of the cells.

Cell debris was removed by centrifugation for 10 min at 8000g, and 15% chloroform was added to the phage-containing supernatant to clear the sample from unprecipitated debris.

This step was found very efficient to decrease microfluidic (chip) and macrofluidic (catheters) problems within the biacore machine.

The catheters of the machine were changed in between rounds of testing.

The phage samples were further purified by PEG - precipitation (phages:PEG 6000, 50% sol., 1:6 v/v), and finally dissolved in HBS-N.

Phage titers following amplification were determined as described above.

### Example 9: Affinity tests of the selected phage populations

To evaluate the binding of phage populations selected from a surface displayed library, affinity tests were performed within the SPR device.

The L1 sensor chip was covered either with VDAC-liposomes or blank liposomes, and blocked (see figure 4).

Coverage of the chip surface was kept high, typically around 7000 RU for VDAC-liposomes and 8000 RU for liposomes.

Amplified phage populations at a concentration of 3,3 x 1010 pfu/ml were injected separately over the two surfaces at high flow rate (30 µl/min) for 2 minutes, after which dissociation curves were followed for 10 minutes.

Prior each injection of phages, a buffer-only control run was performed.

### Example 10: Single clone Amplification

A portion of the top agarose of individual plaques was scraped up and used to infect 10 ml BLT5615 cells as described above.

### Example 11: PCR amplification of single clones

5 µl of the amplified individual phages were dispersed in 100 µl EDTA (10 mM, pH 8).

The samples were vortexed, heated for 10 min at 65°C and cooled to RT.

After a 10 min centrifugation period (14000g) the phage lysates were added to the PCR mix (Taq PCR Mastermix, Qiagen) with appropriate primers.

The PCR conditions after a hot start (10 min at 80°C) were 94°C for 50 sec, 50°C for 1 min, and 72°C for 1 min (35 cycles), followed by 6 min at 72°C.

Finally the samples were purified (Qiaquick purification kit) and controlled by electrophoresis on a 1% agarose gel.

### Example 12: Cycle Sequencing

To analyse the insert sequences in the individual phages, the PCR products were amplified and colour-tagged using the ABI Prism® dGTP BigDyeTM Terminator Ready Reaction Kit (Applied Biosystems).

Afterwards the samples were analysed on an ABI Prism® 377 Automated Sequencer from PERKIN-ELMER.

Briefly, the PCR fragments were combined with a dNTP mix with 1% coloured ddNTP's, appropriate T7 primers and buffer. 25 cycles were performed (96°C for 10 sec, 50°C for 5 sec and 60°C for 4 min). Non-bound dd/dNTP's were separated using Centri-Sep columns (Princeton Separations) and samples concentrated by desiccation.

### Example 13: Cytochrome c oxidase assay

Functional effects of VDAC on COX were assayed by following the oxidation of reduced Cyt c at 550 nm by COX.

Cyt c was brought to its fully reduced state with sodium dithionite (22:1, w/w) in a buffer without detergent (Tris-HCl 10 mM, pH 7.0, KCl 120 mM).

VDAC was dissolved in- 50 mM KCl, 1 mM EDTA, 10 mM Hepes, 1% Triton X-100 by gel filtration and further dilutions were made in the same buffer.

Fixed volumes of the VDAC dilutions were added to fixed volumes of COX (in 20 mM HEPES, 0.1% Dodecyl-Maltoside) and stabilized on ice for 10 min.

This mix was added to fully reduced Cyt c with final reagents concentrations of 0.2 µM COX, 109 µM Cyt c and VDAC ranging from 30 nM to 1 µM.

The final buffer composition was 9.6 mM Tris-HCl, 0.635 mM HEPES, 116 mM KCl, 0.023 mM EDTA and a constant detergent concentration of 0.025 % (0.002 % DM, 0.023 % Triton- X100).

### Example 14: VDAC permeability assay

VDAC-liposomes (LUV) were prepared as described in example 2 except for the last dilution step (1:2, vol/vol) in 0,75 M KCl, and stabilized on ice for 30 min.

Fixed volumes of phages (2.109-2.1010 phage particles, controlled by titration) where added to 8 nmoles of proteoliposome solution, the mix was stabilized for 10 min, and 10 µl of a 200 mM stock of PEG 800 (final conc. 10 mM) was added.

Volume changes were recorded as variations in light scattering with a Biorad® microplate reader at 400 nm.

### Example 15: Clone identification

The DNA insert of one of the positive clones yielded a 224 bp sequence. This sequence perfectly matched a portion of the Homo Sapiens mitochondrial genome (bp 6209 to 6423) coding the cytochrome c oxidase subunit I (Anderson et al. Nature 290, 457-465 (1981)) and corresponding to the region between amino-acids 103 to 172 (figure 7).

From the crystal-structure of the chain A of the bovine heart cytochrome c oxidase (region is 93% identical in human and bovine isoforms) it can be derived that the binding domain spans a β-turn region and its two adjoining α-helices (helices 6 and 7).

### Example 16: Binding of pure COX to pure VDAC

In order to evidence a direct interaction between COX and the channel protein, the binding of the bovine holoenzyme (crystal grade) was measured against pure yeast VDAC reconstructed into proteoliposomes and fixed on the surface of a sensor chip.

For each dose of the holoenzyme, the binding for VDAC-liposomes was preferred against pure liposomes.

### Example 17: Functional effects of VDAC on COX

The binding between these two mitochondrial proteins was never reported before, but hinted at a probable functional interaction.

Purified yeast VDAC enhanced the oxidation rate of Cyt c by the enzyme in a dose-dependent manner.

Initial enzymatic velocities were increased by a factor of 3.5 by nano- to micromolar YVDAC (from 400 to 1400 mOD/min) (figure 8a).

Identical curves were obtained whenever VDAC was pre-incubated with COX or with Cyt c.

Alternatively the slightest variations in detergent concentration markedly altered the oxidation rate. As this dependence on detergent conditions is known, the detergent concentration was kept constant at all the tested VDAC concentrations.

The spontaneous oxidation of Cyt c in these assay conditions was also controlled and found to be 1% per hour.

From the data shown in figure 8a, assuming a one-to-one interaction between the two proteins, a KD value of 200 nM can be derived for VDAC (inset).

When initial velocities are plotted as a function of various substrate concentrations for different VDAC levels (figure 8b) it can be shown that the enzymatic Km values increased with VDAC concentrations.

However, the ratio between the maximal velocity (Vmax) -obtained by hyperbolic fit of the data- and the Km values remained nearly constant (see Table 1). This is further evidenced by the parallel plots on a Lineweaver-Burk chart (figure 9b). Such data are consistent with VDAC modifying the enzyme kinetics in an uncompetitive manner.

**Table 1: Bovine COX kinetic parameter following VDAC addition.**

| | V_{MAX} (µM/min) | K_{M} (µM) | V_{MAX} / K_{M} (1/min) |
|---|---|---|---|
| No VDAC | 125,2 | 33,5 | 3,7 |
| VDAC 50 nM | 132,5 | 28 | 4,7 |
| VDAC 100 nM | 198,7 | 44 | 4,5 |
| VDAC 200 nM | 224,3 | 59 | 3,8 |

### Example 18: Effect of COX on VDAC

VDAC channel function can be assayed by measuring volume changes in proteoliposomes. VDAC-containing liposomes display a re-swelling following the addition of an osmoticant molecule like polyethylene glycol (PEG) if the latter can freely permeate through the channel. This is not the case of pure liposomes or if the channel is closed.

Light scattering -acquired by a spectrophotometer- can follow such changes in liposome volumes.

Figure 6 shows the responses of the proteoliposomes after the addition of PEG to the cuvette (figure 6a). PEG 1500 is not permeable whereas PEG 800 is. The proteoliposomes response following the addition of PEG 800 in the presence of either the naïve phage library or the COX-bearing phage clone is markedly different (figure 6b).

It can be observed that the re-swelling slope is decreased and, this is consistent with a decreased VDAC channel permeability for the osmoticant.

## Claims

1. A method for screening and selection of/for ligands, wherein the target is immobilized on a suitable support, and is any molecule susceptible of binding with said ligands, comprising the step of providing conditions of mass-transfer-limitation, wherein the ligand availability is limited with respect to the target by working under the diffusion limit and wherein the said mass-transfer-limitation conditions are provided by a continuous flow of ligands over said immobilized target.

2. The method according to claim 1, wherein said conditions of mass-transfer-limitation are provided during the biopanning round(s).

3. The method according to any of claims 1 or 2, wherein said mass-transfer-limited conditions are further provided:
- by raising the mass and/or volume of the ligands, preferably in phage display technology or by immobilizing the ligands on beads; and/or
- by providing a magnetic field, said magnetic field being preferably direct current magnetic field or an alternate current magnetic field; and/or
- by providing a temperature gradient.

4. The method according to any of claims 1 to 3, wherein the ligands are any molecules or macro-molecules.

5. The method according to claim 4, wherein said ligands are sugars, proteins, antigens, antibodies, enzymes, DNA, RNA, hormones, neurotransmitters, cells, viruses or any biological entities.

6. The method according to claim 4, wherein the ligands are phage-displayed peptides.

7. The method according to any of claims 1 to 6, wherein the target is a receptor susceptible of binding with said ligands.

8. The method according to claim 7, wherein said receptor is incorporated into liposomes.

9. The method according to claim 8, wherein said liposomes are immobilized upon a sensor chip.

10. Use of the method according to any of claims 1 to 9 for drug design.

11. Use of the method according to any of claims 1 to 9 for catalyst design.

12. Use of the method according to any of claims 1 to 9 for trapping-ligand design, or molecular scavenger design.

13. The method of Claim 1 or 2 comprising the steps of:
i. preparing a solution containing a library of phage-displayed peptides to be screened,
ii. immobilizing the target membrane protein incorporated into liposomes upon a sensor chip,
iii. perfusing a surface plasmon detector device with said solution, for contacting said phage displayed peptides with said target captured upon said sensor chip via said liposomes,
iv. eluting the phage displayed peptides, and
v. optionally, repeating, at least once, step (i) wherein said solution is any amplified eluted fraction(s) of previous step (iv), optionally step (ii), step (iii), and step (iv).

## Patentansprüche

1. Verfahren zum Screening und zur Auswahl von/für Liganden, wobei das Ziel auf einem geeigneten Träger immobilisiert wird und ein beliebiges Molekül ist, das sich an derartige Liganden binden kann, umfassend den Schritt der Herstellung von Bedingungen für einen beschränkten Stoffübergang, wobei die Verfügbarkeit des Linganden hinsichtlich des Ziels dadurch beschränkt ist, dass unter der Diffusionsgrenze gearbeitet wird, und wobei die Bedingungen beschränkten Stoffübergangs durch einen kontinuierlichen Ligandenfluss über dem immobilisierten Ziel hergestellt werden.

2. Verfahren nach Anspruch 1, wobei die Bedingungen beschränkten Stoffübergangs während der Biopanrunde(n) hergestellt werden.

3. Verfahren nach einem der Ansprüche 1 oder 2, wobei die Bedingungen beschränkten Stoffübergangs ferner hergestellt werden:
- durch Erhöhung der Masse und/oder des Volumens der Liganden, vorzugsweise im Phage-Display-Verfahren oder durch Immobilisierung der Liganden auf Kugeln; und/oder
- durch Herstellung eines Magnetfeldes, welches vorzugsweise ein Gleichstrom-Magnetfeld oder ein Wechselstrom-Magnetfeld ist; und/oder
- durch Herstellung eines Temperaturgradienten.

4. Verfahren nach einem der Ansprüche 1 bis 3, wobei die Liganden beliebige Moleküle oder Makromoleküle sind.

5. Verfahren nach Anspruch 4, wobei es sich bei den Liganden um Zucker, Proteine, Antigene, Antikörper, Enzyme, DNA, RNA, Hormone, Neurotransmitter, Zellen, Viren oder sonstige biologische Einheiten handelt.

6. Verfahren nach Anspruch 4, wobei es sich bei den Liganden um Phage-Display-Peptide handelt.

7. Verfahren nach einem der Ansprüche 1 bis 6, wobei das Ziel ein Rezeptor ist, der sich an die Liganden binden kann.

8. Verfahren nach Anspruch 7, wobei der Rezeptor in Liposome aufgenommen wird.

9. Verfahren nach Anspruch 8, wobei die Liposome auf einem Sensorchip immobilisiert werden.

10. Verwendung des Verfahrens nach einem der Ansprüche 1 bis 9 zur Entwicklung von Arzneimitteln.

11. Verwendung des Verfahrens nach einem der Ansprüche 1 bis 9 zur Entwicklung von Katalysatoren.

12. Verwendung des Verfahrens nach einem der Ansprüche 1 bis 9 zur Entwicklung von Fangliganden oder Molekular-Scavengem.

13. Verfahren nach Anspruch 1 oder 2, umfassend die nachfolgenden Schritte:
i. Herstellung einer Lösung, die eine Bibliothek von Page-Display-Peptiden enthält, die einem Screening zu unterziehen sind,
ii. Immobilisieren des in die Liposome auf einem Sensorchip aufgenommenen Zielmembranproteins,
iii. Durchströmung eines Oberflächen-Plasmonen-Detektors mit der Lösung, um die Phage-Display-Peptide mit dem Ziel in Berührung zu bringen, das auf dem Sensorchip mittels der Liposome gefangen wurde,
iv. Eluieren der Phage-Display-Peptide, und
v. wahlweises mindestens einmaliges Wiederholen des Schritts (i), wobei die Lösung (eine) beliebige verstärkte eluierte Fraktion(en) aus dem vorigen Schritt (iv), wahlweise Schritt (ii), Schritt (iii) und Schritt (iv) ist.

## Revendications

1. Procédé pour cribler et sélectionner des ligands, dans lequel la cible est immobilisée sur un support approprié, et est une quelconque molécule susceptible de se lier auxdits ligands, comprenant l'étape qui consiste à appliquer des conditions de limitation du transfert de masse, où la disponibilité du ligand est limitée par rapport à la cible en travaillant sous la limite de diffusion, et où lesdites conditions de limitation du transfert de masse sont obtenues par un flux de ligands continu sur ladite cible immobilisée.

2. Procédé selon la revendication 1, dans lequel lesdites conditions de limitation du transfert de masse sont appliquées pendant le/les tour(s) de biopanning.

3. Procédé selon l'une quelconque des revendications 1 ou 2, dans lequel lesdites conditions de limitation du transfert de masse sont en outre obtenues :
- en augmentant la masse et/ou le volume des ligands, de préférence dans une technologie d'exposition sur phages ou en immobilisant les ligands sur des billes ; et/ou
- en fournissant un champ magnétique, ledit champ magnétique étant de préférence un champ magnétique à courant continu ou un champ magnétique à courant alternatif ; et/ou
- en fournissant un gradient de température.

4. Procédé selon l'une quelconque des revendications 1 à 3, dans lequel les ligands sont de quelconques molécules ou macromolécules.

5. Procédé selon la revendication 4, dans lequel lesdits ligands sont des sucres, des protéines, des antigènes, des anticorps, des enzymes, de l'ADN, de l'ARN, des hormones, des neurotransmetteurs, des cellules, des virus ou de quelconques entités biologiques.

6. Procédé selon la revendication 4, dans lequel les ligands sont des peptides exposés sur des phages.

7. Procédé selon l'une quelconque des revendications 1 à 6, dans lequel la cible est un récepteur susceptible de se lier auxdits ligands.

8. Procédé selon la revendication 7, dans lequel ledit récepteur est incorporé dans des liposomes.

9. Procédé selon la revendication 8, dans lequel lesdits liposomes sont immobilisés sur une puce de capteur.

10. Utilisation du procédé selon l'une quelconque des revendications 1 à 9 pour la conception d'un médicament.

11. Utilisation du procédé selon l'une quelconque des revendications 1 à 9 pour la conception d'un catalyseur.

12. Utilisation du procédé selon l'une quelconque des revendications 1 à 9 pour la conception d'un ligand de piégeage, ou la conception d'un capteur moléculaire.

13. Procédé selon la revendication 1 ou 2, comprenant les étapes qui consistent à :
i. préparer une solution contenant une banque de peptides exposés sur des phages à cribler,
ii. immobiliser la protéine membranaire cible incorporée dans des liposomes sur une puce de capteur,
iii. perfuser un dispositif de détection de plasmons de surface avec ladite solution, afin de mettre en contact lesdits peptides exposés sur les phages avec ladite cible capturée sur ladite puce de capteur via lesdits liposomes,
iv. éluer les peptides exposés sur les phages, et
v. facultativement, répéter, au moins une fois, l'étape (i) dans laquelle ladite solution est une/de quelconque(s) fraction(s) éluée(s) amplifiée(s) de l'étape (iv) précédente, facultativement l'étape (ii), l'étape (iii), et l'étape (iv).


### SEQUENCE LISTING

<110> Vrije Universiteit Brussel
<120> Method for screening and selecting ligands
<130> BP.VUBB.018B/EWD
<160> 2
<170> PatentIn version 3.3
<210> 1
   <211> 215
   <212> DNA
   <213> Homo sapiens
<400> 1
   tctgactctt acctccctct ctcctactcc tgctcgcatc tgctatagtg gaggccggag     60
   caggaacagg ttgaacagtc taccctccct tagcagggaa ctactcccac cctggagcct    120
   ccgtagacct aaccatcttc tccttacacc tagcaggtgt ctcctctatc ttaggggcca    180
   tcaacttcat cacaacaatt atcaatataa aaccc                               215
<210> 2
   <211> 70
   <212> PRT
   <213> Homo sapiens
<400> 2
   Trp Leu Leu Pro Pro Ser Leu Leu Leu Leu Leu Ala Ser Ala Met Val
   1               5                   10                  15
   Glu Ala Gly Ala Gly Thr Gly Trp Thr Val Tyr Pro Pro Leu Ala Gly
               20                  25                  30
   Asn Tyr Ser His Pro Gly Ala Ser Val Asp Leu Thr Ile Phe Ser Leu
           35                  40                  45
   His Leu Ala Gly Val Ser Ser Ile Leu Gly Ala Ile Asn Phe Ile Thr
       50                  55                  60
   Thr Ile Ile Asn Met Lys
   65                  70